Europäisches Patentamt

European Patent Office

Office européen des brevets

(11) Publication number: **0 345 362 B1**

## EUROPEAN PATENT SPECIFICATION

(45) Date of publication of patent specification: **05.08.92**

(51) Int. Cl.⁵: **A61K 7/48**, A61K 31/34, A61K 31/70

(21) Application number: **88107813.3**

(22) Date of filing: **16.05.88**

(54) Use of 2,4-monofurfurylidene-sorbitol and its tetraalkyl derivatives in cosmetics and dermatology.

(30) Priority: **28.05.87 IT 2070787**

(43) Date of publication of application:
**13.12.89 Bulletin 89/50**

(45) Publication of the grant of the patent:
**05.08.92 Bulletin 92/32**

(84) Designated Contracting States:
**AT BE CH DE ES FR GB GR IT LI LU NL SE**

(56) References cited:
**GB-A- 1 091 134**
**GB-A- 1 427 479**

**CHEMICAL ABSTRACTS, vol. 97, no. 21, November 22, 1982, Columbus, Ohio, USA P. ZANOLI et al.: "Influence of 2,4-tetra-O-methyl-furfurylidene-sorbitol (MSF) on carrageean-induced inflammation and antiinflammatory and toxic effects of indomethacin in rats" p. 50, column 1, abstract no. 174 654j & Agents Actions 1982, 12 (4) 521-6**

(73) Proprietor: **LABORATORI FITOCOSMESI E FARMACEUTICI SRL**
**Viale Bianca Maria 33**
**Milano(IT)**

(72) Inventor: **Bertelli, Vittorio**
**Via Carducci 32**
**Milano(IT)**

(74) Representative: **Gervasi, Gemma**
**NOTARBARTOLO & GERVASI Srl Viale**
**Bianca Maria 33**
**I-20122 Milan(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 345 362 B1

**Description**

This invention relates to the use of 2,4-monofurfurylidene-sorbitol and its tetraalkyl derivatives in the preparation of cosmetics and dermatology compositions.

More particularly, the invention relates to the use of 2,4-monofurfurylidene-sorbitol and 2,4-monofurfurylidene-1,3,5,6-0-tetra-alkyl-sorbitol wherein the alkyls are linear or branched $C_1$-$C_4$ in the preparation of cosmetics and dermatology compositions for preventing premature ageing of the skin.

Skin ageing, resulting in the loss of elasticity, the formation of dark spots and the appearance of lines, is caused mainly by the free radicals which form either physiologically by metabolic processes in the skyn cells, or by the action of solar ionizing or atomic radiation or of pollutant substances in the atmosphere or environment.

Atmospheric pollutants include in particular the fumes from industrial chimneys and the exhaust gas of internal combustion engines;
environmental pollutants include in particular tobacco smoke;
and ultraviolet rays constitute the main harmful factor of solar radiation.

Said pollutant substances and said radiation increase the formation of free radicals to thus accelerate the skin ageing process, and in addition can cause serious harm such as skin tumours.

It is well known that free radicals are very reactive unstable chemical structures which give rise to chain reactions (L.B. Myers, Fed. Proc: 32, 1882 (1973)). If the random agents are polycyolic hydrocarbons contained in the industrial fumes or engine exhaust gas, or X-rays, gamma-rays, UV-rays or higher-frequency visible radiation, the reaction chaincommences during exposure by the effect of the supplied energy which is transferred to an electron by the Compton or photoelectric effect. The electron travels within the medium with the formation of ionized molecules which produce highly reactive radicals.

These radicals exert a basic influence on the skin ageing processes by reaction with the proteins, lipids and nucleic acids of the cutaneous cells.

2,4-monofurfurylidene-sorbitol is known to be a substance suitable for pharmaceutical use, such as in the treatment of hepatic and intestinal disturbances (U.S.A. patent 3,383,279).

We have now found that the application of 2,4-monofurfurylidene-sorbitol and its tetraalkyl derivatives of the present invention prevent the formation of free radicals either of endogenous or exogenous origin, and also arrest their action if they existed before the application.

The existence of free radicals is shown by ESR signals, and these are easily detected in the skin after exposure to the aforesaid agents.

Thus the compounds of the present invention can be advantageously used in cosmetics and dermatology for preventing or eliminating the effects of free radicals.

The action mechanism of said compounds is different from that of vitamins E, A, K and C in that these possess only antioxidant action, and not antiradical action.

The compounds of the invention are prepared for topical application in various compositions such as creams, gels, lotions, sprays, etc. Topical application of these compositions prevents cutaneous damage caused by radiation and by the atmospheric agents which produce the free radicals in the skin which cause premature ageing.

Said compositions have a content of the invention compounds of between 0,5 and 2% by weight and preferably between 0,7 and 1,5% by weight and comprise the usual components known to the cosmetics art such as vaseline oil, glycols, triethanolamine, perfume etc.

The composition is prepared under hot conditions while stirring.

The preparation temperature is between 60 and 85°C and preferably between 70 and 75°C, the composition then being left to cool to ambient temperature while stirring.

The following examples of cosmetics and dermatology compositions comprising compounds of the present invention for the prevention of skin ageing are given hereinafter by way of illustration.

EXAMPLE 1

| Preparation of an O/W cream | % by weight |
|---|---|
| A) Beeswax/PEG 8 (APIFIL-GATEFOSSE) | 8 |
| Vaseline oil | 15 |
| Polypropylene glycol | 7 |
| B) Demineralized water | 67,8 |
| 2,4-monofurfurylidene-sorbitol | 1 |
| Methylchloroisothiazolinone and | |
| Methylisothiazolinone (ROHM AND HAAS C.) | 0,1 |
| Carbomer 934 (T.M. GOODRICH) | 0,3 |
| Sodium dehydroacetate | 0,2 |
| C) Triethanolamine (50% solution) | 0,6 |
| Perfume | as required |
| | ----------- |
| Total | 100 |

A) The mixture of beewax, vaseline oil and propylene glycol is melted.
B) The water is heated to 70°C, the 2,4-monofurfurylidene-sorbitol and the other components are added and the mixture left to stand.
A) and B) are heated separately to 75°C after which B) is poured into A) while stirring.
C) The TEA solution is added, the mixture cooled to 35°C while stirring and the perfume added.

EXAMPLE 2

| Preparation of a  W/O cream | % by weight |
|---|---|
| A) Copolymer (22) ethylene oxide and | |
| dodecylglycol (Elfacos ST37 = AKZO CHEMIE) | 3 |
| BHT (butylhydroxytoluene) | 0,02 |
| Hydroxyoctacosanyl hydroxystearate | 5 |
| Vaseline oil | 10 |
| Isoacetylstearate | 10 |
| Copolymer of methoxy (22) ehtylene oxide and | |
| dodecylglycol (Elfacos E200 AKZO CHEMIE) | 3 |
| B) Demineralized water | 62,88 |
| 2,4-monofurfurylidene-sorbitol | 0,8 |
| Sodium dehydroacetate | 0,2 |
| Sorbitol 70 | 5 |
| C) Perfume | as required |
| | ----------- |
| Total | 100 |

A) The compound mixture is melted while stirring
B) The water is heated to 70°C, the 2,4-monofurfurylidene-sorbitol is dissolved and the other components then added.

The mixtures A) and B) are heated separately to 75°C and B) is then poured into A) while stirring. The mixture is cooled to 35°C while stirring and the perfume C) added.

EXAMPLE 3

| Preparation of a gel | % by weight |
|---|---|
| Demineralized water | 87,5 |
| 2,4 monofurfurylidene-sorbitol | 1 |
| Carbomer 940 (T.M. GOODRICH) | 0,5 |
| Sorbitol 70 | 5 |
| Glycerin | 5 |
| 50% triethanolamine solution | 1 |
| Sodium dehydroacetate | 0,2 |
| Perfume | as required |
| Total | 100 |

The 2,4-monofurfurylidene-sorbitol is dissolved in the water heated to 70°C, the carbomer is dispersed, the dispersion allowed to stand, and the following added in the stated order while stirring: sorbitol, glycerin, the TEA solution, the sodium dehydroacetate and the perfume.

EXAMPLE 4

| Preparation of a W/S cream | % by weight |
|---|---|
| A) Abil WE-09 (TEGO-GOLDSCHMIT) | 5 |
| Caprilic-caprinic tryglycerid | |
| (Mygliol 812-DYNAMIT NOBEL) | 11 |
| PCL liquid (DRAGOCO A.G.) | 6 |
| Perfume | as required |
| B) Demineralized water | 72,5 |
| Sodium cloride | 2 |
| 2,4-monofurfurylidene-1,2,5,6-O-tetra- | |
| methylsorbitol | 0,75 |
| Glycerin | 3 |
| Total | 100 |

A) The components Abil, Mygliol, PCL and the perfume are mixed.
B) Sodium chloride, 2,4-monofurfurylidene-1,3,5,6-O-tetramethylsorbitol are dissolved in the demineraliz-

5

ed water.
The mixture B) is poured into the mixture A) while strongly stirring. Stirring is continued for 15 minutes.

EXAMPLE 5

| Preparation of a W/S milk | % by weight |
|---|---|
| A) Abil WS-08 (TEGO-GOLDSCHMIT) | 5 |
| Vaseline oil | 17 |
| Mygliol 812 (DYNAMIT NOBEL) | 10 |
| Perfume | as required |
| B) Demineralized water | 58,8 |
| Sodium chloride | 2 |
| 2,4-monofurfurylidene-1,3,5,6-0-tetrapropyl-sorbitol | 1,2 |
| Glycerin | 3 |
| Sorbitol | 3 |
| Total | 100 |

A) The components Abil, vaseline oil, Mygliol and the perfume are mixed.
B) Sodium chloride, 2,4-monofurfurylidene-1,3,5,6-0-tetrapropylsorbitol, glycerin and sorbitol are added to the demineralized water.
The mixture B) is poured into the mixture A) while strongly stirring. Stirring is continued for 15 minutes.

EXAMPLE 6

| Preparation of a W/O cream | % by weight |
|---|---|
| A) Glyceryl monostearate (ESPERIS) | 10 |
| Vaseline | 10 |
| Vaseline oil | 10 |
| Beeswax | 5 |
| Lanolin | 25 |

6

```
          Preservatives                                      0,15

      B) Demineralized water                                 39

          2,4 monofurfurylidene-1,3,5,6-0-tetrabutyl-

          sorbitol                                           0,85

      C) Perfume                                        as required
                                                        -----------
                                          Total            100
```

A) The mixture of glyceryl monostearate, vaseline, vaseline oil, beeswax, lanoline and preservatives is melted at 75°C.
B) The 2,4-monofurfurylidene-1,3,5,6-0 is added to the demineralized water heated to 72°C.
The mixture B) is poured into the mixture A) while stirring. When the temperature decreases to 35°C perfume is added.

EXAMPLE 7

```
      Preparation of a 0/W cream                       % by weight

      A) Beeswax                                             5

          Arlacel 60 (ATLAS)                                 3

          Tween 60 (ATLAS)                                   4

          Hydrogenated vegetable oil (PROCTER & GAMBLE)    17,5

          Vaseline light oil                                26

          Preservatives and antioxidants                   0,2

      B) Demineralized water                              38,5

          2,4-monofurfurylidene-1,3,5,6-0-tetra-n-

          butylsorbitol                                      1

          Sorbitol                                           5


          Citric acid                                      0,1

          Preservatives                                   0,15
                                                        -----------
                                          Total            100
```

A) The mixture of beeswax, Arlacel, Tween, vegetable oil, vaselin oil, preservatives and antioxidants is melted.
B) The 2,4-monofurfurylidene-1,3,5,6-0-tetra-n-butylsorbitol, citric acid, preservatives and antioxidants are dissolved in the dimineralized water heated to 70°C.

The mixtures A) and B) are heated separately to 75°C and B) is then poured into A) while stirring.

The mixture is cooled to 35°C whyle stirring and a perfume is added.

EXAMPLE 8

```
Preparation of an antisolar hydroalcoholic lotion     % by weight

A) Glyceryl-p-aminobenzoate (ESCALOL VAN DYK)              3

   Propilenglycol ricinoleate                             10

   Glycerin                                               10

   Ethyl alcohol                                          64

   Perfume                                          as required

B) Demineralized water                                    12

   2,4-monofurfurylidene-1,3,5,6-0-tetra-pro-

   pylsorbitol                                             1
                                                  -----------
                                       Total             100
```

A) The components A) are mixed.

B) The 2,4-monofurfurylidene-1,3,5,6,-0-tetrapropylsorbitol is dissolved in the demineralized water.

The solution B) is added to the mixture A) and the new mixture is submitted to filter press.

## Claims

**Claims for the following Contracting States : AT, BE, CH, DE, FR, GB, IT, LI, LU, NL, SE**

1. The use of 2,4-monofurfurylidene-sorbitol and 2,4monofurfurylidene-1,3,5,6-0-tetraalkylsorbitol wherein the alkyls are linear or branched $C_1$-$C_4$, in the preparation of cosmetics and dermatology compositions for preventing premature ageing of the skin caused by free radicals whether formed physiologically or by the action of radiation or of pollutant substances in the atmosphere or environment.

2. Cosmetics and dermatology compositions for preventing skin ageing, comprising between 0.5 and 2% by weight and preferably between 0,7 and 1,5% by weight of 2,4-monofurfurylidene-sorbitol or 2,4 -monofurfurylidene-1,3,5,6-0-tetraalkylsorbitol wherein the alkyls are linear or branched $C_1$-$C_4$.

3. Compositions as claimed in Claim 2, characterised by being in the form of creams, gels, lotions or sprays.

4. Compositions as claimed in Claim 2, characterised by comprising in addition to the 2,4-monofurfurylidene-sorbitol and 2,4-monofurfurylidene-1,3,5,6-0-tetraalkylsorbitol wherein the alkyls are linear or branched $C_1$-$C_4$, other components generally used in cosmetics such as vaseline oil, glycols, triethanolamine and perfume.

5. Compositions as claimed in claims 2 to 4, characterised by being formed by mixing the components together at a temperature of between 60 and 85°C and preferably between 70 and 75°C and allowing the mixture to cool to ambient temperature while stirring.

**Claims for the following Contracting States : ES, GR**

1. Process for preparing cosmetic and dermatologic compositions for cutaneous topic application, in form of creams, gels, lotions or sprays, which comprises mixing conventional vehicles and excipients with 2,4-monofurfurylidene-sorbitol and/or 2,4-monofurfurylidene-1,3,5,6-O-tetraalkylsorbitol, through stirring at temperature between 60° and 85°C and allowing the mixture to cool to room temperature while stirring.

2. Process according to claim 1 wherein the amount of 2,4-monofurfurylidene-sorbitoland/or 2,4-monofurfurylidene-1,3,5,6-O-tetraalkylsorbitol added to the mixture is comprised between 0,5% and 2% by weight on the total.

**Revendications**
**Revendications pour les Etats contractants suivants : AT, BE, CH, DE, FR, GB, IT, LI, LU, NL, SE**

1. Utilisation de 2,4-monofurfurylidène-sorbitol et de 2,4-monofurfurylidène-1,3,5,6-O-tétraalkylsorbitol, dans lequel les alkyles en $C_1$ à $C_4$ sont linéaires ou ramifiés, dans la préparation de compositions cosmétiques et dermatologiques, en vue d'empêcher le vieillissement prématuré de la peau causé par les radicaux libres dont la formation est un résultat physiologique ou s'effectue sous l'action de rayonnements ou de substances polluantes de l'atmosphère ou de l'environnement.

2. Compositions cosmétiques et dermatologiques en vue d'empêcher le vieillissement de la peau, comprenant : entre 0,5 et 2 % en poids et, de préférence, entre 0,7 et 1,5 % en poids de 2,4-monofurfurylidène-sorbitol ou de 2,4-monofurfurylidène-1,3,5,6-O-tétraalkylsorbitol dans lequel les alkyles en $C_1$ à $C_4$ sont linéaires ou ramifiés.

3. Compositions telles que revendiquées dans la revendication 2, caratérisées par le fait qu'elles sont sous forme de crèmes, de gels, de lotions ou de pulvérisations.

4. Compositions telles que revendiquées dans la revendication 2, caractérisées par le fait qu'elles comprennent, outre le 2,4-monofurfurylidène-sorbitol et du 2,4-monofurfurylidène-1,3,5,6-O-tétraalkylsorbitol dans lequel les alkyles en $C_1$ à $C_4$ sont linéaires ou ramifiés, d'autres composants généralement utilisés dans le domaine de la cosmétique, tels que de l'huile de vaseline, des glycols, la triéthanolamine et du parfum.

5. Compositions telles que revendiquées dans les revendications 2 à 4, caractérisées par le fait qu'elles sont formées en mélangeant ensemble les composants à une température comprise entre 60° C et 85° C et, de préférence, entre 70° C et 75° C, puis en laissant le mélange refroidir à température ambiante tout en agitant.

**Revendications pour les Etats contractants suivants : ES, GR**

1. Procédé de préparation de compositions cosmétiques ou dermatologiques, en vue d'une application topique cutanée, sous forme de crèmes, de gels, de lotions ou de pulvérisations, qui comprend le mélange de véhicules et d'excipients courants avec le 2,4-monofurfurylidène-sorbitol et/ou du 2,4-monofurfurylidène-1,3,5,6-O-tétraalkylsorbitol, tout en maintenant sous agitation à une température comprise entre 60° C et 85° C et en laissant refroidir le mélange à température ambiante tout en agitant.

2. Procédé selon la revendication 1, dans lequel la quantité de 2,4-monofurfurylidène-sorbitol et/ou de 2,4-monofurfurylidènel-1,3,5,6-O-tétraalkylsorbitol ajouté(s) au mélange est comprise entre 0,5 % et 2 % en poids du total.

**Patentansprüche**
**Patentansprüche für folgende Vertragsstaaten : AT, BE, CH, DE, FR, GB, IT, LI, LU, NL, SE**

1. Verwendung von 2,4-Monofurfurylidensorbitol und 2,4-Monofurfuryliden-1,3,5,6-O-tetraalkylsorbitol, worin die Alkylgruppen lineare oder verzweigte $C_1$-$C_4$-Alkylgruppen sind, bei der Herstellung von Kosmetika und dermatologischen Zusammensetzungen zur Verhinderung einer vorzeitigen Alterung der Haut, hervorgerufen durch freie Radikale, die entweder physiologisch oder durch Einwirkung von Strahlung oder von Schadstoffen aus der Atmosphäre oder der Umwelt gebildet werden.

2. Kosmetika und dermatologische Zusammensetzungen zur Verhinderung der Hautalterung, enthaltend 0,5 bis 2 Gew.%, vorzugsweise 0,7 bis 1,5 Gew.%, 2,4-Monofurfurylidensorbitol oder 2,4-Monofurfuryliden-1,3,5,6-O-tetraalkylsorbitol, worin die Alkylgruppen lineare oder verzweigte $C_1$-$C_4$-Alkylgruppen sind.

3. Zusammensetzungen gemäß Anspruch 2,
dadurch **gekennzeichnet,** daß
sie in der Form von Crèmes, Gelen, Lotionen oder Sprays vorliegen.

4. Zusammensetzungen gemäß Anspruch 2,
dadurch **gekennzeichnet,** daß
sie zusätzlich zum 2,4-Monofurfurylidensorbitol und 2,4-Monofurfuryliden-1,3,5,6-O-tetraalkylsorbitol, worin die Alkylgruppen lineare oder verzweigte $C_1$-$C_4$-Alkylgruppen sind, weitere in Kosmetika allgemein verwendete Bestandteile wie Vaselineöl, Glycole, Triethanolamin und Parfüm enthalten.

5. Zusammensetzungen gemäß Ansprüchen 2 bis 4,
dadurch **gekennzeichnet,** daß
sie gebildet werden, indem man die Komponenten bei einer Temperatur von 60 bis 85°C, vorzugsweie 70 bis 75°C, zusammenmischt und die Mischung unter Rühren auf Umgebungstemperatur abkühlen läßt.

**Patentansprüche für folgende Vertragsstaaten : ES, GR**

1. Verfahren zur Herstellung kosmetischer und dermatologischer Zusammensetzungen zur cutanen topischen Aufbringung, in Form von Crèmes, Gelen, Lotionen oder Sprays, wobei man herkömmliche Träger und Exzipienten mit 2,4-Monofurfurylidensorbitol und/oder 2,4-Monofurfuryliden-1,3,5,6-O-tetraalkylsorbitol durch Rühren bei einer Temperatur von 60 bis 85°C vermischt und die Mischung unter Rühren auf Raumtemperatur abkühlen läßt.

2. Verfahren gemäß Anspruch 1,
worin die der Mischung zugefügte Menge an 2,4-Monofurfurylidensorbitol und/oder 2,4-Monofurfuryliden-1,3,5,6-O-tetraalkylsorbitol 0,5 bis 2 Gew.%, bezogen auf Gesamtgewicht, beträgt.